# EUROPÄISCHE PATENTSCHRIFT

(11) **EP 2 698 134 B1**
(45) Veröffentlichungstag und Bekanntmachung des Hinweises auf die Patenterteilung: **03.06.2015**
(21) Anmeldenummer: 13176300.5
(22) Anmeldetag: 12.07.2013
(51) Int. Cl.: A61F 9/00

(54) **Tropfenspender**
Drop dispenser
Distributeur compte-gouttes

(30) Priorität: 14.08.2012 DE 102012214426
(43) Veröffentlichungstag der Anmeldung: 19.02.2014
(73) Patentinhaber: Aptar Radolfzell GmbH, 78315 Radolfzell (DE)
(72) Erfinder: Wochele, Matthias, 78239 Rielasingen-Worblingen (DE); Greiner-Perth, Jürgen, 78244 Gottmadingen (DE)
(74) Vertreter: Patentanwälte Ruff, Wilhelm, Beier, Dauster & Partner

(56) Entgegenhaltungen:
- EP-A1- 0 222 944
- WO-A1-84/00707
- US-A- 6 105 828

## Beschreibung

Die Erfindung betrifft einen Flüssigkeitsspender zur Abgabe von Tropfen mit einem Gehäuse, mit einem Flüssigkeitsreservoir, mit einer Austragöffnung, durch die die Flüssigkeit aus dem Flüssigkeitsreservoir ausgetragen werden kann, und mit einer die Austragöffnung umgebenden Tropfenbildungsfläche, an der sich die Flüssigkeit sammelt, um von der Tropfenbildungsfläche als Tropfen abgegeben zu werden.

Gattungsgemäße als Tropfenspender ausgebildete Flüssigkeitsspender sind aus dem Stand der Technik bekannt, so beispielsweise aus der DE 102010048085 A1 und der DE 102008001313 B3.

Bei gattungsgemäßen Tropfenspendern ist als nahezu unvermeidbares Problem festzustellen, dass mit der Abgabe eines Tropfens, des Haupttropfens, an der Tropfenbildungsfläche ein Rest, der so genannte Resttropfen, verbleibt, der nachfolgend entweder an der Tropfenbildungsfläche trocknet oder aber wieder in den Tropfenspender eingesogen wird. Dieser Resttropfen stellt eine Gefahr dar, da er sowohl an der Tropfenbildungsfläche als auch im Flüssigkeitsreservoir eine Kontamination verursachen kann.

Es ist daher gewünscht, den Resttropfen hinsichtlich seines Volumens soweit als möglich zu minimieren, damit die Kontamination, insbesondere beim Trocknen des Tropfens an der Tropfenbildungsfläche, gering gehalten wird. Ein kleinerer Resttropfen führt zu einem schnelleren Trocknen desselben und damit zu einer verringerten Kontaminationsgefahr.

Dem genannten Problem versucht bereits die nachveröffentlichte DE 102011083355 A1 Herr zu werden, indem hier eine Geometrie der Tropfenbildungsfläche vorgeschlagen wird, die den Resttropfen zum Teil ausfüllt und dadurch das Flüssigkeitsvolumen des Resttropfens verringert.

Weiterhin ist aus der EP 0222944 A1 bekannt, einen Tropfenspender mit einem elastischen Auslassschlauch zu versehen, der einenschlitzartigen Auslass umfasst. Weitere im Kontext der Erfindung als Hintergrund nennenswerte Dokumente sind die WO 84/00707 A1 und die US 6,105,828 A.

### Aufgabe und Lösung

Aufgabe der Erfindung ist es, eine hierzu alternative oder ergänzende Möglichkeit zur Verfügung zu stellen, um das Volumen des Resttropfens zu verringern.

Erfindungsgemäß wird dies dadurch erreicht, dass die Tropfenbildungsfläche eine äußere Begrenzungskontur aufweist, deren Formgebung von einer Kreisform abweicht, wobei zumindest Teilabschnitte der Begrenzungskontur Einbuchtungen bilden, die konkav nach innen und somit zur Austragöffnung hin gewölbt sind.

Der erfindungsgemäße Tropfenspender weist übereinstimmend mit gattungsgemäßen Tropfenspendern ein Gehäuse auf, welches das Flüssigkeitsreservoir beherbergt und aus dem durch die Austragöffnung hindurch die Flüssigkeit zur Tropfenbildungsfläche gefördert werden kann. Grundsätzlich kommen verschiedene Arten von Fördermechanismen hierfür in Frage. Insbesondere ist bei gattungsgemäßen sowie erfindungsgemäßen Tropfenspendern vorgesehen, dass die das Flüssigkeitsreservoir umgebenden Wandungen des Gehäuses verformbar sind, sodass durch unmittelbare Kraftbeaufschlagung des Gehäuses, welches als Quetschflasche agiert, das Volumen des Flüssigkeitsreservoirs vermindert werden kann. Grundsätzlich sind jedoch auch andere Mechanismen wie beispielsweise eine Kolbenpumpe denkbar und von der Erfindung umfasst.

Die genannte Tropfenbildungsfläche eines erfindungsgemäßen sowie eines gattungsgemäßen Tropfenspenders ist jene außenseitig der Austragöffnung vorgesehene Fläche, die beim Austrag von Flüssigkeit durch die Austragöffnung hindurch vollständig benetzt wird und jenseits derer an der Außenseite des Gehäuses eine Benetzung nicht stattfindet. Wodurch die äußere Begrenzungskontur der Tropfenbildungsfläche definiert wird, wird im Weiteren noch erläutert.

Erfindungsgemäß ist diese Begrenzungskontur bezogen auf eine Blickrichtung in Richtung der durch die Austragöffnung definierten Austragachse nicht kreisrund.

Es hat sich gezeigt, dass eine von der üblichen kreisrunden Form der Begrenzungskontur abweichende Formgebung die Flüssigkeitsmenge des abzugebenden Haupttropfens bei vergleichbarer Größe der Tropfenbildungsfläche kaum beeinflusst, die Größe des an der Tropfenbildungsfläche nach der Abgabe des Haupttropfens verbleibenden Resttropfens jedoch deutlich jedoch reduziert.

Somit kann durch eine Änderung der Formgebung der Tropfenbildungsfläche auf sehr einfache konstruktive Art das Problem des Resttropfens deutlich verringert werden.

Die Größe der Tropfenbildungfläche bzw. der durch die Begrenzungskontur umschlossenen Fläche hängt von der Größe der zu erzeugenden

Tropfen ab. Als besonders vorteilhaft wird es angesehen, wenn die von der Begrenzungkontur umschlossene Fläche, welche gemeinsam durch die Tropfenbildungsfläche und die Querschnittsfläche der Auslassöffnung gebildet wird, eine Größe zwischen 1 mm² und 5 mm² quadrat aufweist, insbesondere eine Größe zwischen 1,5 mm² und 2,5 mm². Hierdurch lassen sich Tropfen im Bereich von etwa 35µl bis 40µl erzeugen.

Die genannte Begrenzungskontur, die die Tropfenbildungsfläche nach außen begrenzt und die für die im Tropfenspender enthaltene Flüssigkeit jene Linie darstellt, bis zu der sich die Flüssigkeit jenseits der Austragöffnung sammelt, bevor sie als Haupttropfen abgegeben wird, kann auf verschiedene Art und Weise definiert werden.

Besonders von Vorteil ist es, wenn die Begrenzungskontur durch eine scharfe Kante gebildet wird, an der die plane oder nur leicht gewölbte Tropfenbildungsfläche übergeht in einen benachbarten konischen oder zylindrischen Gehäuseabschnitt. Um zu verhindern, dass die Flüssigkeit über diese Kante hinwegströmt, weist die Kante vorzugsweise einen Krümmungsradius von weniger als 0,2 mm, insbesondere weniger als 0,1mm, auf sowie insbesondere vorteilhafterweise eine Kantenwinkel von mindestens 30° auf.

Eine andere, jedoch vorteilhafterweise gemeinsam mit einer solchen scharfkantigen Begrenzungskante vorgesehene Möglichkeit zur Definition der Begrenzungslinie sieht vor, dass die Tropfenbildungsfläche bis zur Begrenzungslinie eine verglichen mit umgebenden Gehäuseteilen hydrophilere Oberflächenbeschaffenheit aufweist. Die Flüssigkeit benetzt im Zuge der Tropfenbildung in einem solchen Falle aufgrund der vergleichsweise hydrophoben Gestaltung außerhalb der Begrenzungskontur nur die innerhalb der Begrenzungskontur angeordnete Tropfenbildungsfläche. Die unterschiedliche Hydrophilie lässt sich durch Oberflächen der vorzugsweise aus Kunststoff gefertigten Außenflächen des Spenders bewerkstelligen. Auch der Aufbau des Spenders bzw. von dessen Austragkopf aus mehreren Teilen aus unterschiedlichem Kunststoff kann den gewünschten Sprung in der Hydrophilie bewirken.

Vorzugsweise weicht die Formgebung der Tropfenbildungsfläche deutlich von der Kreisform ab. Hierunter wird verstanden, dass die Begrenzungskontur teilweise von der durch die Austragsöffnung definierten Austragachse um mindestens Faktor 1,2, vorzugsweise um mindestens Faktor 1,5, weiter entfernt ist als von einem von der Austragachse minimal beabstandeten Teil der Begrenzungskontur.

Weiterhin ist die von der Begrenzungskontur umschlossene Fläche vorzugsweise mindestens 10% kleiner als die Innenfläche eines Kreises, dessen Durchmesser der maximalen Erstreckung der von der Begrenzungskontur umgebenen Fläche entspricht. Vorzugsweise ist diese Fläche sogar um mindestens 15%, insbesondere vorzugsweise um mindestens 20% kleiner als eine entsprechende Kreisfläche.

Wenngleich bereits eine einzige Einbuchtung der Begrenzungskontur einen solchen Effekt in messbarem Maße verursacht, ist es von Vorteil, wenn eine Mehrzahl von Einbuchtungen, vorzugsweise zwischen zwei und sechs Einbuchtungen, insbesondere sechs Einbuchtungen vorgesehen sind.

Die Einbuchtungen sind vorzugsweise gleichmäßig über den Umfang verteilt.

In einfachster Ausgestaltung ist die Tropfenbildungsfläche plan. Grundsätzlich sind aber auch konkave und konvexe Gestaltungen zweckmäßig. Insbesondere eine konvexe Gestaltung mit nach außen leicht gewölbter Tropfenbildungsfläche ist geeignet, das Volumen des verbleibenden Resttropfens nochmals zu reduzieren. Die Wölbung der Tropfenbildungsfläche ist hier so beschaffen, dass ein Normalenvektor stets mit der Austragachse der Austragöffnung einen Winkel <15° einschließt. Besonders bevorzugt ist die Verwendung einer Tropfenbildungsfläche beschriebener Art bei Tropfenspendern, bei denen ein Auslassventil vorgesehen ist, welches den Rückfluss der Flüssigkeit in das Flüssigkeitsreservoir verhindert. Gerade bei Spendern mit einem solchen Auslassventil ist die Kontamination des an der Außenfläche verbleibenden Resttropfens besonders nachteilig.

Der erfindungsgemäße Spender ist insbesondere für pharmazeutische Flüssigkeiten, insbesondere für ophtalmische Flüssigkeiten, vorgesehen und somit im Lieferzustand hiermit befüllt. Insbesondere handelt es sich um pharmazeutische Flüssigkeiten zur Behandlung des erhöhten Augeninnendrucks (Glaukombehandlung), zur Behandlung des trockenen Auges und zur Behandlung von Allergien und Entzündungen. Hierbei spielen insbesondere die Molekülgruppen Alpha-2 Agonisten, z. B. Brimonidin, Prostaglandinanaloga (Tafluprost, Latanoprost, Bimatoprost, Travoprost), Beta-Blocker, z. B. Timolol, und Carboanhydrasehemmer, z. B. Dorzolamid beziehungsweise Hyaluronsäureverbindungen, Filmbildner, z. B. Methylcelluloseverbindungen und Cyclosporin beziehungsweise Antihistaminika, z. B. Olopatadin und Levocabastin, Steroide, z. B. Loteprdnol und Dexamethason, sowie NSAID, z. B. Keterolac, eine Rolle.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches anhand der Figuren nachfolgend erläutert wird. Dabei zeigen:
- Figur 1: einen erfindungsgemäßen Tropfenspender in einer Gesamtdarstellung,
- Figuren 2 und 3: den Austragkopf des Spenders gemäß Figur 1 in einer teilgeschnittenen Seitenansicht und einer Draufsicht und
- Figuren 4a bis 4c: eine Tropfenbildungsfläche des Spenders der Figuren 1 bis 3 sowie zwei alternative Formgebungen einer solchen Tropfenbildungsfläche.

### Detaillierte Beschreibung der Ausführungsbeispiele

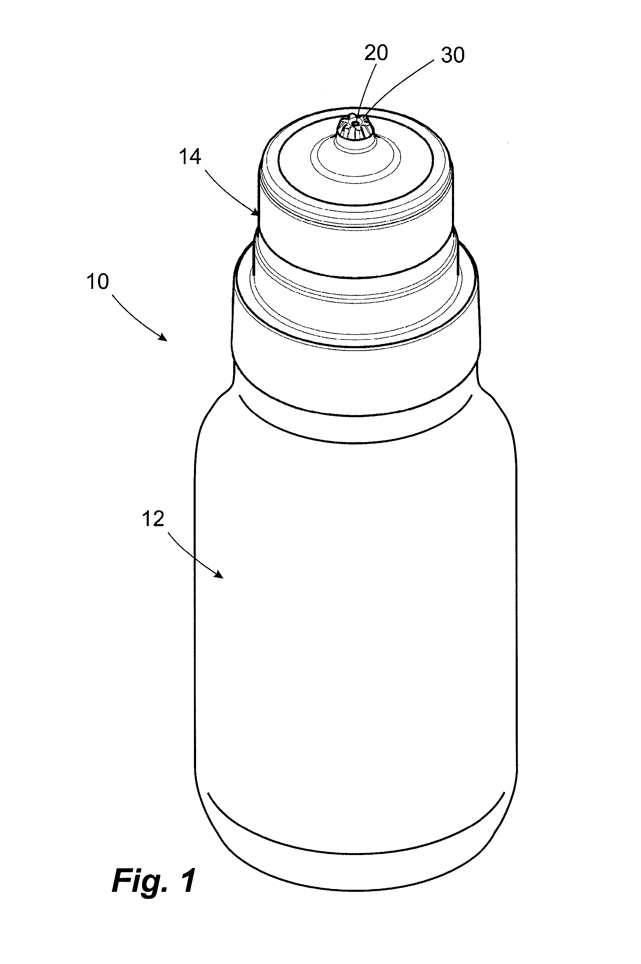

Figur 1 zeigt einen erfindungsgemäßen Tropfenspender 10 in einer Gesamtdarstellung. Dieser Tropfenspender 10 verfügt über ein Gehäuse, welches durch einen Flaschenkörper 12, der die Außenwandung eines Flüssigkeitsreservoirs darstellt, und einen Austragkopf 14 gebildet ist.

Am distalen Ende des Austragkopfs 14 ist eine Austragöffnung 20 vorgesehen, die von einer Tropfenbildungsfläche 30 umgeben wird.

Die erfindungsgemäße Verwendung des Spenders 10 der Figur 1 sieht vor, dass dieser in einer Lage mit nach unten weisender Austragsöffnung 20 durch Kraftbeaufschlagung des Flaschenkörpers 12 in Art einer Quetschflasche zusammengedrückt wird, wobei die Volumenreduzierung des Flüssigkeitsspeichers dazu führt, dass Flüssigkeit durch die Austragöffnung 20 hindurch gefördert wird und sich an der Tropfenbildungsfläche 30 ansammelt. Mit fortschreitender Volumenreduktion des Flaschenkörpers 12 während der Betätigung sammelt sich die Flüssigkeit fortgesetzt an der Tropfenbildungsfläche 30, bis sie sich zum überwiegenden Teil in Form eines Haupttropfens von der Tropfenbildungsfläche 30 löst. An der Tropfenbildungsfläche 30 verbleibt ein Resttropfen.

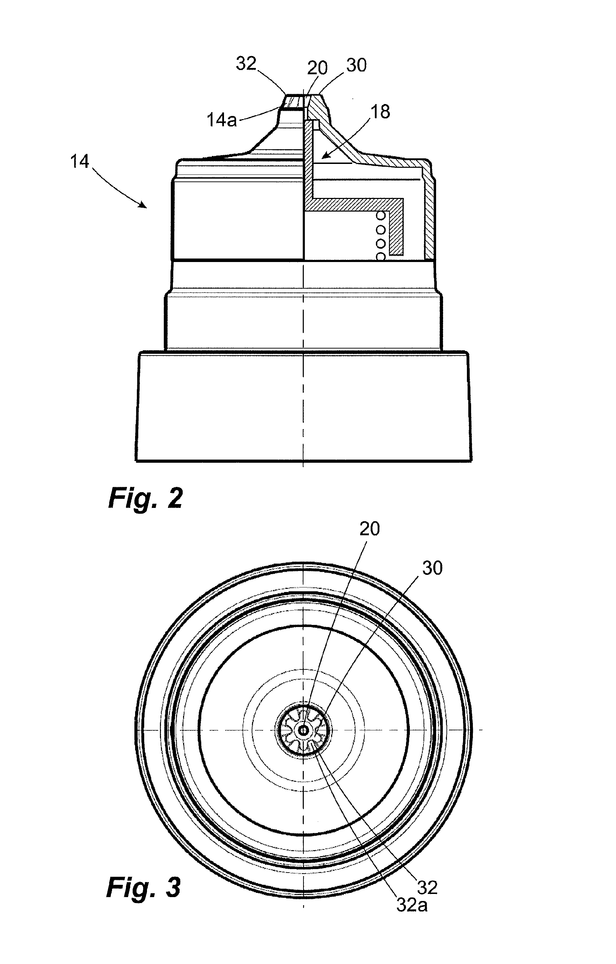

Aufgrund eines federbeaufschlagten Auslassventils 18, welches stark schematisiert in Figur 2 dargestellt ist, kann die verbliebene Flüssigkeitsmenge des Resttropfens nicht in das Flüssigkeitsreservoir zurückkehren. Sie verbleibt stattdessen außenseitig an der Tropfenbildungsfläche 30 und trocknet dort.

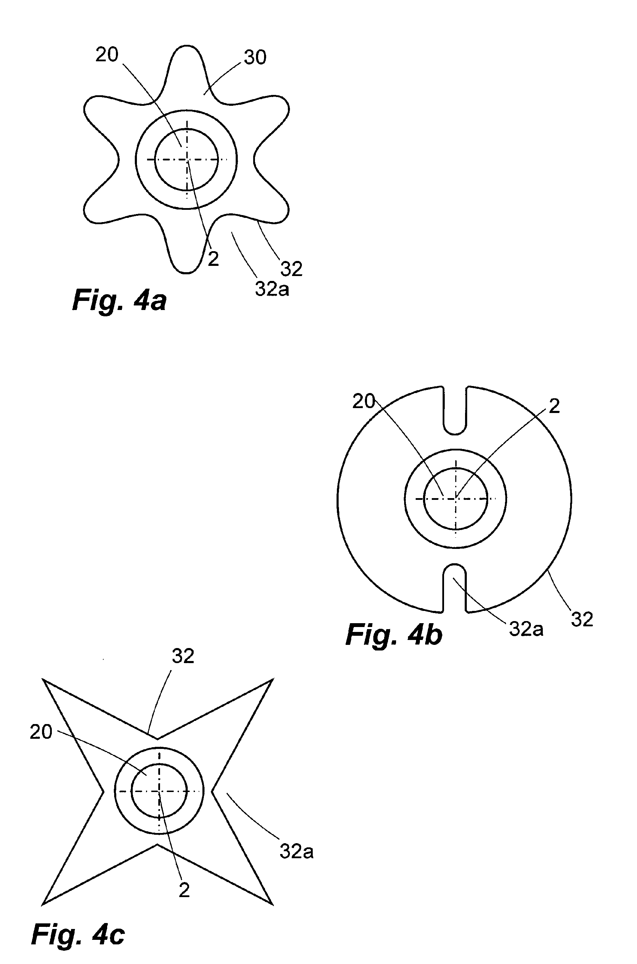

Die Tropfenbildungsfläche weist eine Formgebung auf, die diesen Trocknungsprozess beschleunigt, indem sie bewirkt, dass das Volumen des Reststropfens nach Ablösen des Haupttropfens vergleichsweise klein bleibt. Die Formgebung der Tropfenbildungsfläche ist in Figur 3 und insbesondere auch in Figur 4a zu erkennen. Diese Tropfenbildungsfläche 30 ist außenseitig durch eine Begrenzungskontur 32 begrenzt, welche eine scharfe Kante zu sich daran anschließenden konischen Gehäuseaußenflächen 14a bildet. Diese Begrenzungskontur 32 ist im Falle des Spenders der Figuren 1 bis 3 und 4a mit insgesamt sechs Einbuchtungen 32a versehen und weist somit in etwa die Form eines sechsstrahligen Sterns oder eine sechsblättrigen Blume auf. Verglichen mit einer kreisrunden Tropfenbildungsfläche gleicher Größe wird die Tropfenbildung des Haupttropfens durch diese Geometrie kaum beeinflusst.

Versuche haben gezeigt, dass die Reduzierung des Tropfenvolumens gegenüber einer gleichgroßen kreisförmigen Tropfenbildungsfläche bei unter 10% liegt. Dies kann durch eine gegebenenfalls etwas größere Fläche der Tropfenbildungsfläche 30 kompensiert werden. Die Versuche haben allerdings auch gezeigt, dass das Volumen des verbleibenden Resttropfens nach Abgabe eines Tropfens drastisch reduziert ist. Der Resttropfen, der an der Tropfenbildungsfläche 30 gemäß dem Spender der Figuren 1 bis 3 und 4a verbleibt, ist nur noch etwa halb so groß wie ein Resttropfen, der an einer kreisrunden Tropfenbildungsfläche gleicher Fläche verbliebe.

Somit ist auf einfache Art und Weise das Problem des Resttropfens deutlich reduziert. Konkret ist der Spender der Fig. 1 bis 3 in der Lage, Haupttropfen eines Volumens von mehr als 40µl zu erzeugen, während der dabei verbleibende Resttropfen ein Volumen von weniger als 4µl aufweist. Dies ist ein sehr gutes Volumenverhältnis.

Die Figuren 4b und 4c zeigen alternative Formgebungen einer Tropfenbildungsfläche 30. Diese Tropfenbildungsflächen stimmen mit der Tropfenbildungsfläche der Figur 4a insoweit überein, als dass ebenfalls die maximal beabstandeten Teile der Begrenzungskontur deutlich weiter von einer Austragachse 2 beabstandet sind als die minimal hiervon beabstandeten Teile.

Die Ausgestaltung gemäß der Figur 4c sieht eine sternförmige Form mit vier von der Austragöffnung 20 wegweisenden Strahlen vor, zwischen denen entsprechend geformte Einbuchtungen 32a vorgesehen sind. Im Falle der Figur 4b weist die Tropfenbildungsfläche 30 lediglich an zwei gegenüberliegenden Seiten Einbuchtungen auf.

Die genannten Formgebungen der Figuren 4b und 4c führen zu einer signifikanten Volumenreduzierung des Resttropfens. Bei einer jeweiligen Dimensionierung der Tropfenbildungsflächen der Figuren 4b und 4c derart, dass der hierdurch gebildete Haupttropfen gleich groß wie bei einer kreisrunden Tropfenbildungsfläche ist, reduziert sich das Volumen des Resttropfens zwischen etwa 20 % (Tropfenbildungsflächen der Figur 4b) bis zu 50 % (Tropfenbildungsfläche der Figur 4a).

Für alle Tropfenbildungsflächen 30 der Fig. 4a bis 4c gilt, dass diese durch eine scharfe Kante, wie in Fig. 1 bis 3 dargestellt, und/oder durch einen Sprung in der Hydrophilie der Oberflächen innerhalb und außerhalb der Begrenzungskonturen 32 begrenzt sein können.

## Patentansprüche

1. Flüssigkeitsspender (10) zur Abgabe von Tropfen mit
- einem Gehäuse (12, 14),
- einem Flüssigkeitsreservoir (12),
- einer Austragöffnung (20), durch die Flüssigkeit aus dem Flüssigkeitsreservoir (12) ausgetragen werden kann, und
- einer die Austragöffnung 812) umgebenden Tropfenbildungsfläche (30), an der sich die Flüssigkeit sammelt, um von der Tropfenbildungsfläche (30) als Tropfen abgegeben zu werden,
wobei die Tropfenbildungsfläche (30) eine äußere Begrenzungskontur (32) aufweist, deren Formgebung von einer Kreisform abweicht, **dadurch gekennzeichnet, dass** zumindest Teilabschnitte der Begrenzungskontur (32) Einbuchtungen (32a) bilden, die konkav nach innen gewölbt sind.

2. Flüssigkeitsspender (10) nach Anspruch 1,
**dadurch gekennzeichnet, dass**
die Begrenzungskontur (32) der Tropfenbildungsfläche (30) durch eine Begrenzungskante mit einem Krümmungsradius von weniger als 0,1 mm gebildet wird.

3. Flüssigkeitsspender (10) nach Anspruch 1 oder 2,
**dadurch gekennzeichnet, dass**
die Begrenzungskontur (32) der Tropfenbildungsfläche (30) dadurch gebildet ist, dass innerhalb der Begrenzungskontur (32) die Tropfenbildungsfläche (30) hydrophiler als die umgebende Außenflächen (14a) des Gehäuses (12, 14) außerhalb der Begrenzungskontur (32) ausgebildet ist.

4. Flüssigkeitsspender (10) nach einem der vorstehenden Ansprüche,
**dadurch gekennzeichnet, dass**
der einer durch die Austragöffnung (20) definierten Austragsachse (2) maximal beabstandete Teil der Begrenzungskontur (32) von der Austragachse (2) um mindestens Faktor 1,2 weiter entfernt ist als der minimal beabstandeten Teil der Begrenzungskontur (32).

5. Flüssigkeitsspender (10) nach einem der vorstehenden Ansprüche,
**dadurch gekennzeichnet, dass**
- die mehreren Einbuchtungen (32a) gleichmäßig über den Umfang der Tropfenbildungsfläche (30) verteilt sind und/oder
- mindestens drei Einbuchtungen (32a) vorgesehen sind.

6. Flüssigkeitsspender (10) nach einem der vorstehenden Ansprüche,
**dadurch gekennzeichnet, dass**
die Tropfenbildungsfläche (30) konkav oder konvex ausgebildet ist.

## Claims

1. A liquid dispenser (10) for dispensing drops, comprising
- a housing (12, 14),
- a liquid reservoir (12),
- a discharge opening (20) through which liquid from the liquid reservoir (12) can be discharged, and
- a drop formation surface (30) surrounding the discharge opening (20), where the liquid accumulates in order to be discharged as a drop from the drop formation surface (30),
wherein the drop formation surface (30) has an outer limiting contour (32), the shape of which deviates from a circular shape,
**characterized in that**
at least partial sections of the limiting contour (32) form notches (32a), which are curved with inward concavity.

2. The liquid dispenser (10) according to claim 1,
**characterized in that**
the limiting contour (32) of the drop formation surface (30) is formed by a limiting edge having a curvature radius of less than 0.1 mm.

3. The liquid dispenser (10) according to claim 1 or 2,
**characterized in that**
the limiting contour (32) of the drop formation surface (30) is formed **in that** within the limiting contour (32), the drop formation surface (30) is formed more hydrophilic than the surrounding external faces (14a) of the housing (12, 14) outside of the limiting contour (32).

4. The liquid dispenser (10) according to any one of the preceding claims,
**characterized in that**
the part of the limiting contour (32) at maximum distance to a discharge axis (2) defined by the discharge opening (20) is more distant from the discharge axis (2) at least by a factor 1.2 than the part of the limiting contour (32) at minimum distance.

5. The liquid dispenser (10) according to any one of the preceding claims, **characterized in that**
- the plurality of notches (32a) are distributed uniformly over the circumference of the drop formation surface (30) and/or
- at least three notches (32a) are provided.

6. The liquid dispenser (10) according to any one of the preceding claims,
**characterized in that**
the drop formation surface (30) is formed concavely or convexly.

## Revendications

1. Distributeur de liquide (10) pour délivrer des gouttes, avec
- un boîtier (12, 14)
- un réservoir de liquide (12),
- une ouverture d'extraction (20), à travers laquelle du liquide peut être extrait hors du réservoir de liquide (12), et
- une face de formation de gouttes (30) entourant l'ouverture d'extraction (20), sur laquelle le liquide s'accumule afin d'être délivré à partir de la face de formation de gouttes (30) sous la forme de gouttes,
dans lequel la face de formation de gouttes (30) présente un contour de limitation extérieur (32), dont la forme s'écarte d'une forme circulaire,
**caractérisé en ce qu'**au moins des régions partielles du contour de limitation (32) forment des enfoncements (32a), qui sont incurvés vers l'intérieur sous forme concave.

2. Distributeur de liquide (10) selon la revendication 1, **caractérisé en ce que** le contour de limitation (32) de la face de formation de gouttes (30) est formé par une arête de limitation présentant un rayon de courbure de moins de 0,1 mm.

3. Distributeur de liquide (10) selon la revendication 1 ou 2, **caractérisé en ce que** le contour de limitation (32) de la face de formation de gouttes (30) est formé par le fait que, à l'intérieur du contour de limitation (32), la face de formation de gouttes (30) est plus hydrophile que les faces extérieures environnantes (14a) du boîtier (12, 14) à l'extérieur du contour de limitation (32).

4. Distributeur de liquide (10) selon l'une quelconque des revendications précédentes, **caractérisé en ce que** la partie du contour de limitation (32) espacée au maximum d'un axe d'extraction (2) défini par l'ouverture d'extraction (20) est plus éloignée d'au moins un facteur 1, 2 de l'axe d'extraction (2) que la partie du contour de limitation (32) espacée au minimum.

5. Distributeur de liquide (10) selon l'une quelconque des revendications précédentes, **caractérisé en ce que**
- les multiples enfoncements (32a) sont répartis uniformément sur la périphérie de la face de formation de gouttes (30), et/ou
- il est prévu au moins trois enfoncements (32a).

6. Distributeur de liquide (10) selon l'une quelconque des revendications précédentes, **caractérisé en ce que** la face de formation de gouttes (30) est de forme concave ou convexe.
